(19) Europäisches Patentamt
European Patent Office
Office européen des brevets

(11) **EP 0 535 317 B1**

(12) **EUROPÄISCHE PATENTSCHRIFT**

(45) Veröffentlichungstag und Bekanntmachung des Hinweises auf die Patenterteilung:
**19.06.1996 Patentblatt 1996/25**

(51) Int. Cl.$^6$: **A61K 35/78**

(21) Anmeldenummer: 92111709.9

(22) Anmeldetag: **09.07.1992**

(54) **Arzneimittel zur Behandlung von Erregungszuständen und Nervendysfunktionen**

Drug for the treatment of agitation state and nerve dysfonction

Médicament pour le traitement de disfonctionnement nerveux et d'états d'excitation

(84) Benannte Vertragsstaaten:
**AT BE CH DE DK ES FR GB GR IT LI LU NL PT SE**

(30) Priorität: **09.07.1991 DE 4122706**

(43) Veröffentlichungstag der Anmeldung:
**07.04.1993 Patentblatt 1993/14**

(73) Patentinhaber: **STEIGERWALD ARZNEIMITTELWERK GMBH D-64295 Darmstadt (DE)**

(72) Erfinder:
• **Schneider, Werner, Dr. rer. nat. W-5400 Koblenz (DE)**
• **Elstner, Erich F., Prof. Dr. W-8031 Gröbenzell (DE)**
• **Kleber, Elisabeth, Dr. W-8042 Oberschleissheim (DE)**

(74) Vertreter: **Kolb, Helga, Dr. Dipl.-Chem. et al Hoffmann, Eitle & Partner, Patentanwälte, Postfach 81 04 20 81904 München (DE)**

(56) Entgegenhaltungen:
FR-M- 9 61M       FR-M- 53 60M

• CHEMICAL ABSTRACTS, vol. 95, 1981, Columbus, Ohio, US, abstract no. 129273g; VON DER DUNK, KLAUS: 'POISONOUS PLANTS IN OUR ENVIRONMENT: BLEEDING HEART DICENTRA SPECTABILIS (L.) LEM., GOLDEN POPPY ESCHSCHOLTZIA CALIFORNICA CHAM., AND LARKSPUN CORYDALIS CAVA L.', Seite 352

**Beschreibung**

Die Erfindung betrifft ein Arzneimittel zur Linderung von Erregungszuständen und Nervendysfunktionen.

Es ist bekannt, dass die Zwischen- und Endprodukte des Catecholamin-Stoffwechsels Einfluss auf den Erregungszustand beim Menschen haben. So wirkt z.B. das Endprodukt Adrenalin als ein Neurotransmitter des adrenergen Nervensystems sowohl auf alpha- wie auch β-Rezeptoren. Als Sympathikomimetikum steigert Adrenalin die Kontraktionskraft des Herzens. Da Adrenalin ausserdem den oxidativen Stoffwechsel in den Zellen steigert, bewirkt es ingesamt eine erhöhte Einsatzbereitschaft des Organismus. Dementsprechend beobachtet man auch eine Steigerung der Adrenalinausschüttung in Stress-Situationen. Besonders hohe Konzentrationen von Adrenalin sowie auch dessen Vorstufe, Noradrenalin, können Übererregung, Nervosität und damit mangelnde Leistungsfähigkeit des Körpers zur Folge haben, während nur wenig erhöhte Werte dieser Amine durchaus positiv wirken: Pulsschlag und Blutdruck sind leicht erhöht, die Muskulatur und das Gehirn sind angeregt, die Aufnahmefähigkeit ist gesteigert.

Ausserdem ist bekannt, dass Störungen im Konzentrationsniveau von Dopamin, einer weiteren Vorstufe zu Adrenalin, zu Erregungszuständen und Nervendysfuntionen führen können. Insbesondere können bei Dopamin-Mangel Depressionen auftreten. Ein spezielles Dopamin-Mangelsyndrom stellt die Parkinson-Krankheit dar, deren Teilsymptome Akinese, Rigor und Ruhetremor sind. Neuere klinische Ergebnisse sprechen für eine therapeutische Wirksamkeit von Dopa bei der Behandlung des Parkinsonismus; diese Wirkung erscheint aufgrund der beobachteten Dopamin-Verminderung in den Basalganglien verständlich.

Bei einem Dopamin-Mangel ist es somit wünschenswert, auf natürliche Weise die Konzentration dieses Catecholamins zu erhöhen. Dies kann erreicht werden, indem man einerseits die Dopa/Dopamin-Synthese steigert, und andererseits den Dopamin-Abbau unterbindet.

Aufgabe der vorliegenden Erfindung ist es, ein Arzneimittel zur Verfügung zu stellen, welches die Dopamin-Konzentration eines Patienten im natürlichen Stoffwechsel erhöht.

Die vorstehende Aufgabe wird gemäß der Erfindung durch ein Arzneimittel nach Anspruch 1 gelöst, welches dadurch gekennzeichnet ist, daß es als Wirkstoffe durch alkoholische Extraktion (2.5:10) erhaltene Extrakte von Corydalis und Eschscholtzia, gegebenenfalls neben allgemein üblichen Zusätzen, umfaßt, wobei das Verhältnis (Gewicht) der Extrakte Eschscholtzia zu Corydalis im Bereich von 20:1 bis 1:1: liegt.

Zweckmäßige Ausgestaltungen davon sind Gegenstand der Ansprüche 2 bis 9.

Gegenstand der Erfindung ist ferner die Verwendung eines durch alkoholische Extraktion (2.5:10) erhaltenen Extraktes zur zur Herstellung eines Arzneimittels zur Behandlung von Erregungszuständen und Nervendysfunktionen, die auf einer Störung des Aminhaushaltes beruhen, gemäß Anspruch 10 Verwendungsformen davon sind Gegenstand der Ansprüche 11 bis 13.

Das erfindungsgemäße Arzneimittel kann die durch alkoholische Extraktion erhaltene Extrakte in Form eines gefriergetrockheten Pulvers, einer wässerigen und/oder einer alkoholischen Lösung enthalten. Die Herstellung des gefriergetrockheten Pulvers erfolgt in der Regel durch Gefriertrockhung des durch alkoholische Extraktion erhaltenen Extraktes (Drogenauszuges) nach hierfür an sich üblichen Gefriertrochnungs-Verfahren.

Die gefriergetrockneten Extrakte von Corydalis und Eschscholtzia können getrennt oder in Mischung aufbewahrt werden, und gegebenenfalls zur Herstellung des Arzneimittels mit einer geeigneten pharmazeutischen Trägersubstanz/Verdünner vermischt werden. Zur Herstellung von Tinkturen eignen sich hierfür insbesondere wasser und/oder Alkohol. In erster Linie enthält das Arzneimittel die Wirkstoffe in Form des durch alkoholische Extraktion (2.5:10) erhaltenen Drogenauszugs, und insbesondere besteht es aus einem solchen Drogenauszug.

Das Verhältnis von Eschscholtzia zu Corydalis (bezogen auf das Gewicht des Trockenextraktes) liegt vorzugsweise im Bereich von 10:1 bis 2:1, und beträgt insbesondere 4:1.

Als Wirkstoff werden erfindungsgemäß vorzugsweise Extrakte von Corydalidis cavae rhizoma und Eschscholtziae californicae herba eingesetzt.

Die erfindungsgemäßen Arzneimittel können in einer für eine orale Darreichung üblichen Form vorliegen, insbesondere in Form von Tabletten, Dragees, Kapseln oder in erster Linie Tinkturen. Die Herstellung dieser Darreichungsformen erfolgt, gegebenenfalls unter Verwendung der für die einzelnen Darreichungsformen üblichen und geeigneten pharmazeutischen Verdünnungs- und/oder Trägerstoffe, auf an sich übliche Weise, z.B. durch Tablettieren, Dragieren, Verkapselung des in gefriergetrockneter Form vorliegenden Extraktes oder eine alkoholischen und/oder wässerigen Lösung davon. Zur Herstellung flüssiger Darreichungsformen, wie insbesondere von Tinkturen, werden vorzugsweise die durch die alkoholische Extraktion erhaltenen alkoholischen Drogenauszüge direkt verwendet. Neben der Gefriertrocknung dieser alkoholischen Drogenauszüge zur Herstellung der gefriergetrockneten Extrakte ist es auch möglich, die alkoholischen Drogenauszüge direkt, vorzugsweise im Vakuum und/oder in einer Inertgasatmosphäre, zur Trockene einzudampfen, und den so erhaltenen Rückstand dann in die gewünschte Darreichungsform weiterzuverarbeiten.

Die Konzentration der Wirkstoffe im erfindungsgemäßen Arzneimittel ist in weiten Grenzen variierbar. In der Regel beträgt die Konzentration 0.5 bis 20 Gew. %, bezogen auf die fertige Arzneimittelzusammensetzung, und insbesondere 1 bis 10 Gew. %, in erster Linie 1 bis 4 Gew. %. Wenn der durch alkoholische Extraktion (2.5:10) erhaltene alkoholische Drogenauszug verwendet wird, so wird dieser vorzugsweise in der direkt nach der Extraktion erhaltenen Konzentration eingesetzt; dabei werden die Drogenauszüge aus Corydalidis und Eschscholtziae insbesondere in einem solchen Verhältnis verwendet, daß das Verhältnis von Corydalis zu Eschscholtzia den vorstehend bevorzugt genannten Verhältnissen entspricht.

Zur Herstellung der Einzelextrakte von Corydalis und Eschscholtzia, insbesondere von Corydalis cavae rhizoma und Eschscholtziae californicae herba, werden Drogen eingesetzt, die den Spezifikationen des DAB 9 entsprechen. Dies wird durch eine Qualitätkontrolle bei Erhalt der Droge sichergestellt.

Die Herstellung der einzelnen Extrakte erfolgt in einer an sich bekannten Weise nach hierfür üblichen und standardisierten Extraktionsverfahren, wie z.B. durch Mazeration oder Perkolation mittels Ethanol oder Ethanol/Wasser-Gemischen. Dabei wird ein Drogen-Extrakt-Verhältnis von 2.5:10 eingehalten, d.h., aus 2.5 g Droge werden 10 ml Drogenextrakt gewonnen. Als Extraktionsmittel wird vorzugsweise 30 Vol. %-iges Ethanol eingesetzt. Die Herstellung kann an Hand einer Inprozesskontrolle gesteuert werden.

Zur Beurteilung und Sicherung der gleichbleibenden Qualität der Extrakte werden hierfür übliche Qualitätskriterien herangezogen. Insbesondere dienen dazu die Gesamtspektren der extrahierten Stoffe und andererseits die Analysen der aus der Literatur bekannten Haupt-Alkaloide der beiden Drogen (vgl. z.B. ...).

Die nach der Qualitätskontrolle freigegebenen Extrakte werden dann entsprechend der gewünschten Darreichungsform und Konzentrationen als flüssiger oder gefriergetrockneter Extrakt weiterverarbeitet.

Die Art und Menge der Verabreichung richtet sich insbesondere nach der Schwere der Erkrankung, dem allgemeinen Zustand und dem Alter des Patienten. In der Regel beträgt die Verabreichungsmenge in Form einer Tinktur, die erfindungsgemäß bevorzugt ist, ein bis 2 Tropfen/kg Körpergewicht, ein- oder mehrmals am Tag. In Form anderer oraler Darreichungsformen werden in der Regel entsprechende Mengen verabreicht, vorzugsweise 2 bis 5 mg Gesamttrockkenextrakt/kg Körpergewicht, ein- oder mehrmals am Tag.

Die beiliegenden Figuren haben folgende Bedeutung:

Fig. 1    zeigt das Stoffwechselschema der Catecholamin-Synthese.

Fig. 2    zeigt eine schematische Übersicht des Reaktionsschemas von Tyrosin über Dopa, Dopachinon zu Dopachrom bzw. Melanin.

Fig. 3    zeigt die bei 303 nm UV-spektrometrisch bestimmte Tyrosinhydroxylierung in Anwesenheit von Corydalis-Extrakt 1:10.000, Eschscholtzia-Extrakt 1:10.000 bzw. 20 E Phenolase.

Fig.4    zeigt den Lineweaver-Burk-Plot für die Phenolasereaction mit Corydalis-Extrakt (Fig. 4a und 4b), sowie zum Vergleich ohne Corydalis-Extrakt (Fig. 4c-4f).

Fig. 5    gibt die Phenolasereaktion in Anwesenheit von 3,4-Dihydroxybenzol wieder.

Fig. 6    zeigt den Einfluss von D,L-Dopa auf die Phenolase-Reaktion bei verschiedenen Dopa-Konzentrationen (K = Kontrolle).

Fig. 7    zeigt die Hemmung der Dopamin-$\beta$-hydroxylase durch Eschscholtzia-Extrakt (Kurve A) sowie einer wässerigen 1%-igen Lösung der gefriergetrockneten Extrakte von Corydalis und Eschscholtzia (Kurve B).

Fig. 8    zeigt fotometrisch bei 241 nm die Hemmung der durch eine Aminooxidase katalysierten Umsetzungsreaktion von Benzylamin zu Benzaldehyd durch Corydalis-Extrakt.

Fig. 9    gibt die Hemmung der MAO-katalysierten Benzaldehyd-Bildung bei 241 nm in Abhängigkeit von der Konzentration des Extraktes aus Eschscholtzia wieder.

Fig. 10    zeigt die Hemmung der MAO-katalysierten Benzaldehyd-Bildung bei 241 nm durch Extrakte von Eschscholtzia (A), Corydalis(B) sowie einer Mischung der Extrakte von Corydalis und Eschscholtzia im Verhältnis 20:80.

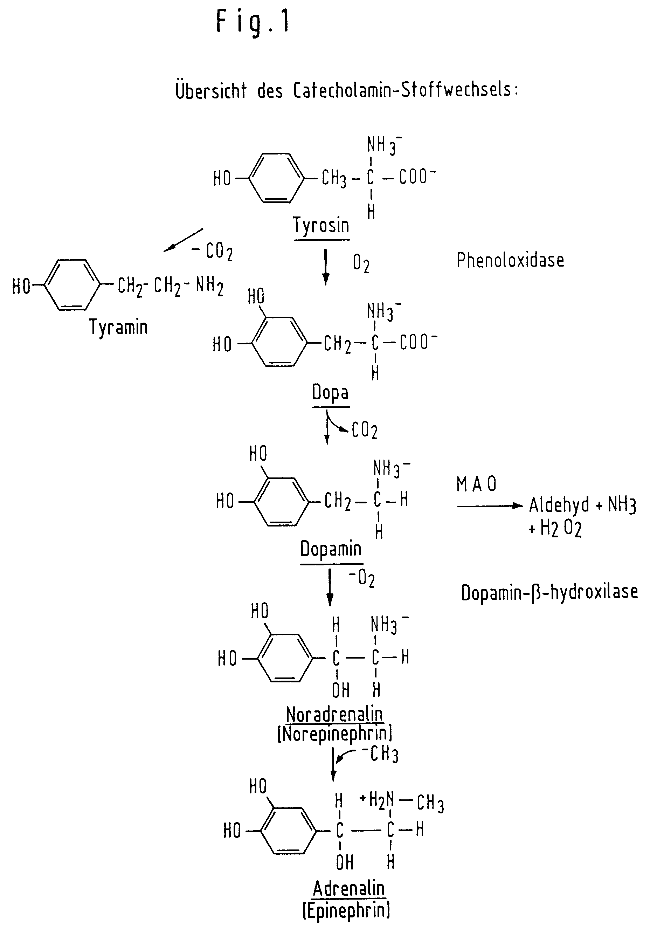

Aus Fig. 1 ist das Stoffwechselschema der Catecholamine ersichtlich. Die dabei auftretenden Verbindungen Dopa und Dopamin sind hinsichtlich ihrer Konzentration einerseits davon abhängig wie schnell die Umwandlung des Tyrosins zu Dopa erfolgt, und andererseits mit welcher Geschwindigkeit Dopamin zu Noradrenalin und Adrenalin weiterreagiert. Ausserdem unterliegt Dopamin auch einem Abbau durch Monoaminooxidasen (MAO), so dass auch hierdurch dessen Konzentration im Stoffwechsel nennenswert beeinflusst werden kann.

Wie Fig. 1 zu entnehmen ist, erhält man eine erhöhte Dopamin-Konzentration, wenn die Reaktion der Phenoloxidase (Phenolase, Tyrosinase) gesteigert, und der Einfluss der Dopamin-β-hydroxylase sowie von Monoaminooxidasen vermindert werden kann.

Im folgenden wurden Experimente durchgeführt, die zeigen, in welchem Masse die aktiven Komponenten des erfindungsgemässen Arzneimittels auf die drei Enzymreaktionen Einfluss nehmen.

Die folgenden Untersuchungen wurden soweit nicht anders angegeben mit im Handel erhältlichen Enzymen und mit Corydalidis cavae rhizoma und Eschscholtziae californicae herba durchgeführt.

**1. Untersuchung der Tyrosinase-Reaktion:**

Tyrosin wird im Stoffwechsel in einer sauerstoffverbrauchenden Reaktion zu L-Dopa hydroxyliert. Diesen ersten Schritt der Catecholamin-Synthese katalysiert eine Tyrosinase bzw. Phenolase oder Phenoloxidase, die ein kupferhaltiges, wenig spezifisches Enzym darstellt.

Es ist bekannt, dass Phenolasen in der Natur weit verbreitet sind, und in Pflanzen, Pilzen und verschiedenen tierischen Geweben vorkommen. Sie spielen überall dort eine wichtige Rolle, wo Mono- oder Diphenole oxidiert werden, wobei in vielen Fällen ozidative Polymerisierungsschritte eingeleitet werden, wie z.B. die Bildung von Lignin oder Melanin.

Phenolasen katalysieren zwei aufeinanderfolgende Oxidationsschritte, die wie folgt beschrieben werden können:

1. Die 'Cresolase-Aktivität' des Enzyms bewirkt die Hydroxylierung eines Monophenols in ortho-Stellung unter Bildung eines Diphenols.

2. Die 'Catecholase-Aktivität' katalysiert die Weiteroxidation des entstandenen o-Diols zum entsprechenden Chinon.

Beide Reaktionen sind unmittelbar voneinander abhängig. Der enge Zusammenhang der Cresolase- und Catecholase-Reaktion beruht auf dem Oxidationsstatus des Kupferatoms im aktiven Zentrum, der nach D. Kertesz und R. Zito in O. Hayaishi (Hrsg.) "Oxygenases", Academic Press, London (1962), Kapitel 8: Phenolase, wie folgt angegeben wird:

$$(Cu^{++})_2\text{-Enzym} + o\text{-Dihydroxyphenol} \rightarrow (Cu^{+})_2\text{-Enzym} + o\text{-Chinon} + 2\ H^{+};$$

$$(Cu^{+})_2\text{-Enzym} + 0{,}5\ O_2 + 2\ H^{+} \rightarrow (Cu^{++})_2\text{-Enzym} + H_2O$$

In der vorstehenden Elektronenbilanz des Kupfers bleibt die Cresolase-Reaktion unberücksichtigt. Tatsächlich scheint nicht geklärt zu sein, ob die Hydroxylierung des Monophenols durch eine echte Enzymreaktion katalysiert wird oder ob diese Reaktion auf chemischem Wege parallel zur Catecholoxidation nach folgendem Schema abläuft:

$$o\text{-Chinon} + Monophenol + H_2O \rightarrow 2\ o\text{-Diphenol}.$$

Für die Hypothese einer nicht-enzymatischen Reaktion spricht vor allem die Tatsache, dass bei der Reaktion von Monophenolen mit Phenolasen eine Induktionsphase zu beobachten ist, die bei Tyrosinasen aus tierischem Gewebe bis zu eine Stunde andauern kann. Eine kontinuierliche Enzymreaktion kann erst dann eintreten, wenn Diole oder Chinone als Verunreinigung zugegen oder durch 'Autoxidation' entstanden sind.

Untersuchung der Tyrosinase-Reaktion mit Pflanzenextrakten aus Corydalis und Eschscholtzia

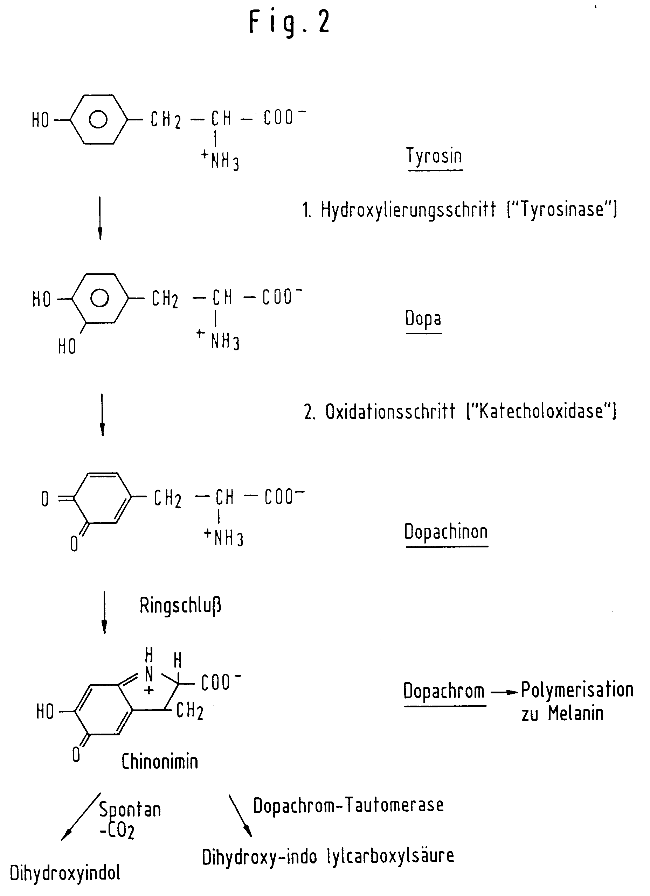

Fig. 2 gibt in schematischer Übersicht das Reaktionsschema, ausgehend von Tyrosin, zu Dopachrom und dessen Umsetzung zu Chinonimin oder Melanin.

Experimentell beobachtet wurde im folgenden die Oxidation von Tyrosin zu Dopachrom (Fig. 2), dem ersten Oxidationsprodukt von Dihydroxyphenylalanin (Dopa). Dopachrom selbst ist ein Substrat für eine 'Dopachrom-Tautomerase' und stellt eine Vorstufe der Melanin-Bildung dar. Sowohl die Bildung von Dopachrom als auch die Melanin-Synthese führen zu einem Abbau von Dopa und damit zu einer verminderten Synthese von Dopamin.

Die Dopachrom-Konzentration kann im UV-Spektrum bei einer Wellenlänge von 303 - 318 nm verfolgt werden. Dabei wird dessen Bildung aus Tyrosin der Aktivität der zu untersuchenden Phenolase, die als geschwindigkeitsbestimmend angesehen wird, gleichgesetzt.

Die Untersuchungen wurden an folgenden Substratgemischen durchgeführt:
2 mM Tyrosin
100 mM Phosphatpuffer, pH 6,5
20 E Tyrosinase oder alkoholische Auszüge von Eschscholtzia bzw. Corydalis, verdünnt im Verhältnis 1:10000 (ausgehend vom Stamm-Extrakt gemäss dem nachfolgenden Beispiel).

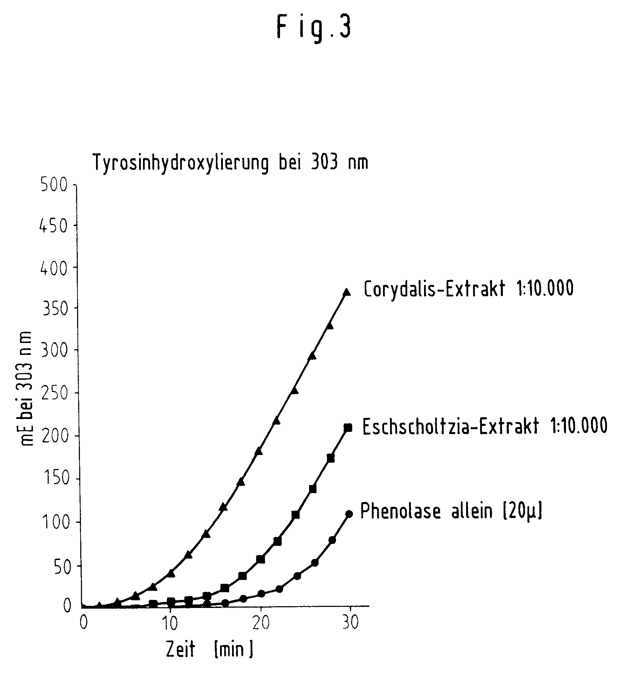

Fig. 3 zeigt die bei 303 nm UV-spektrometrisch bestimmte Tyrosinhydroxylierung in Anwesenheit einer Phenoloxidase bzw. Extrakten aus Corydalis und Eschscholtzia. Dabei beobachtet man in Gegenwart von Phenolase allein das Auftreten einer etwa 15-minütigen 'lag-Phase'.

Verdünnungen aus Corydalis-Extrakten bis 1:10000 führen zu einer deutlichen Verkürzung dieser lag-Phase oder Initialphase, wie dies aus Fig. 3 hervorgeht. Bei höheren Konzentrationen an Corydalis beobachtet man ein sofortiges Einsetzen der maximalen Umsatzrate ohne 'lag-Phase'.

Der Extrakt aus Eschscholtzia führt ebenfalls zu einer verkürzten Initialphase; die Wirksamkeit des Eschscholtzia-Extraktes liegt aber deutlich unter der von Corydalis.

Kontrollversuche haben gezeigt, dass die Anwesenheit entsprechender Mengen an Ethanol keinen Einfluss auf die Reaktion hat. Ausserdem zeigten Versuche mit Pyrrolochinolinchinon (PQQ), dass der Zusatz dieses physiologisch sehr interessanten Chinons keinen Einfluss auf die Initialphase der Phenolase-Reaktion hat. Auch Hypericin, das als para-Chinon eventuell die lag-Phase beeinflussen könnte, ist ohne Einfluss auf die Reaktion.

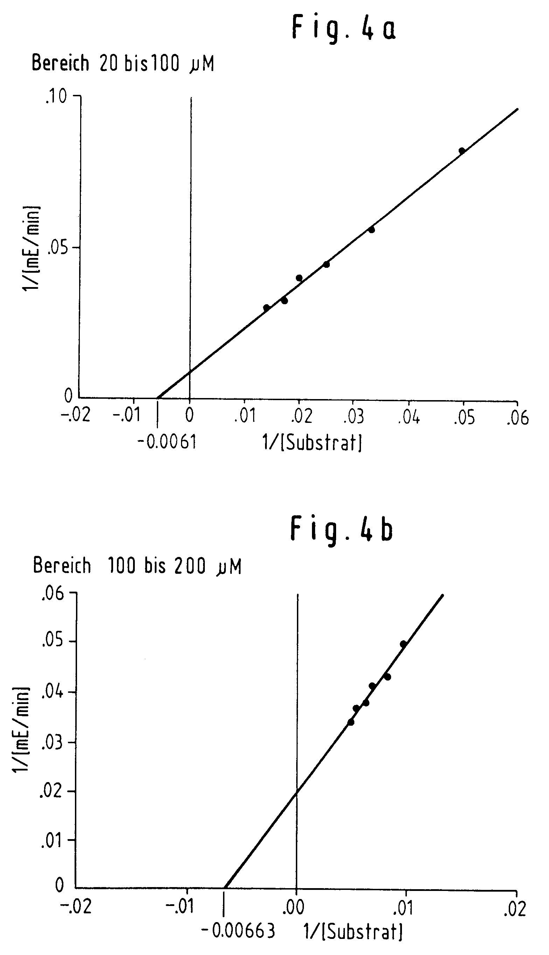

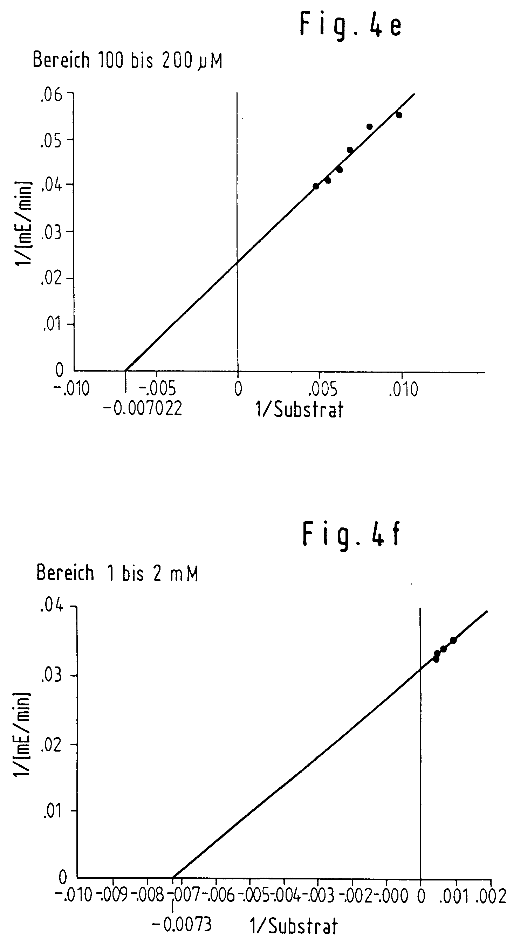

Interessant ist bei dieser Versuchsreihe die Tatsache, dass der Extrakt aus Corydalis zwar den Zeitpunkt der maximalen Umsatzrate von Tyrosin deutlich nach vorn verschiebt, aber keinen Einfluss auf die Maximalumsatzrate hat. Versuche mit unterschiedlichen Substratkonzentrationen zeigen im Lineweaver-Burk-Plot keine Verschiebung des $K_m$-Wertes der Phenolase-Reaktion (siehe hierzu Fig. 4a bis 4f).

Zur weiteren Aufklärung des Mechanismus der Tyrosinase-Reaktion, insbesondere im Hinblick auf eine Verkürzung der lag-Phase, wurden dem Testansatz weitere Substanzen zugegeben:

a) Die Zugabe von Kupfersalzen bewirkte keinen Einfluss auf die Enzymreaktion.

b) Ascorbinsäure als reduzierendes Agens kann - wie dies aus der Literatur bekannt ist - die lag-Phase bei verschiedenen Reaktionstypen unterbinden. Innerhalb der vorliegenden Versuchsreihe konnte dies jedoch nicht beobachtet werden: Ascorbinsäure mag zwar den Umsatz von Tyrosin zu L-Dopa einleiten, verhindert aber gleichzeitig dessen Weiteroxidation zu Dopachrom. Eine Phenolase-Reaktion konnte in Anwesenheit von Ascorbinsäure bei 303 nm über einen Zeitraum von 30 min. nicht beobachtet werden; die 'lag-Phase' war möglicherweise sogar verlängert.

c) Eine Kombination von PQQ mit Ascorbinsäure, wobei die reduzierte Form $PQQH_2$ entsteht, welches als Diol die Reaktion beeinflussen könnte, bewirkte ebenfalls eine Verlängerung der lag-Phase.

d) Die Zugabe 'echter' Diole, wie 3,4-Dihydroxybenzol und D,L-Dopa, verkürzt die lag-Phase der Reaktion (im Falle der Dopa-Zugabe erhöht sich durch das grössere Substratangebot auch die Geschwindigkeit der Dopachrom-Bildung).

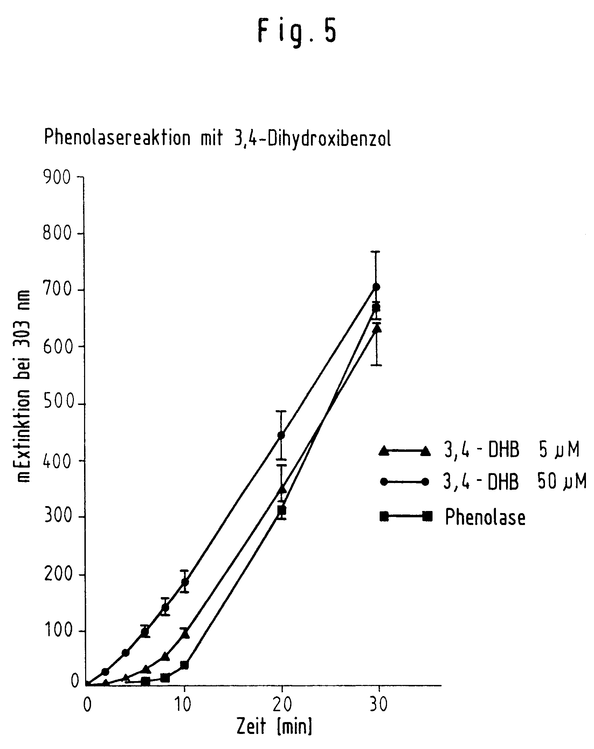

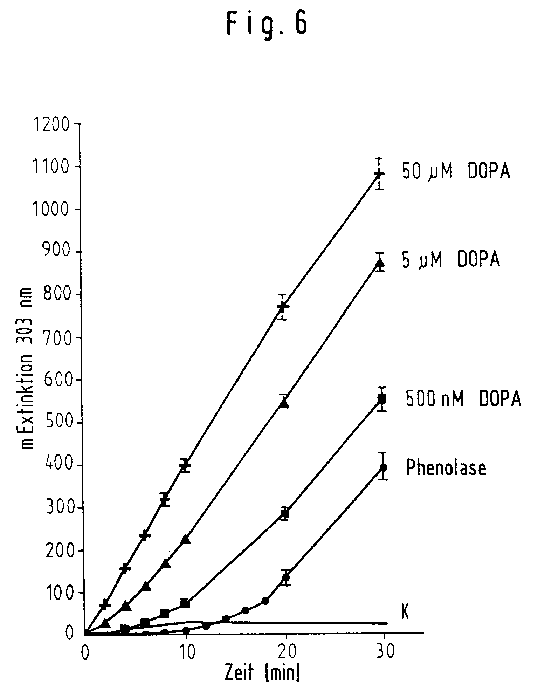

In Fig. 5 wird die Phenolase-Reaktion in Gegenwart von 3,4-Dihydroxybenzol wiedergegeben. Fig. 6 zeigt den Einfluss von D,L-Dopa auf die Phenolase-Reaktion in Abhängigkeit von der Dopa-Konzentration. Sowohl aus Fig. 5 und Fig. 6 kann der Einfluss der genannten Diole auf die lag-Phase deutlich entnommen werden.

Eine Autoxidation von Dopa, welche bei alkalischen pH-Werten auftritt, ist bei pH 6,5 nicht oder nur wenig zu beobachten.

Die vorstehenden Versuche zeigen, dass eine Verkürzung der Initialphase der Phenolase-Reaktion lediglich durch Zugabe von Diolen bewirkt werden kann. Chinoline und Ascorbinsäure zeigen keinen Einfluss. Es ist daher anzunehmen, dass die Extrakte von Corydalis und Eschscholtzia ortho-Diole enthalten, die eine sofortige Umsetzung von Tyrosin zu Dopa bewirken. Es ist im weiteren anzunehmen, dass die Extrakte von Corydalis und Eschscholtzia auch in vivo zu einer schnelleren Umsetzung von Tyrosin und damit zu einer erhöhten Konzentration an Dopa führen.

## 2. Untersuchung der Dopamin-β-hydroxylase-Reaktion:

Das Enzym Dopamin-β-hydroxylase katalysiert die Umsetzung von Dopamin zu Noradrenalin. Dopamin-β-hydroxylase ist ebenfalls ein kupferhaltiges Enzym. Die Hydroylierung erfolgt unter Sauerstoff-Verbrauch und benötigt Ascorbinsäure und Fumarat als reduzierende Agenzien, um den Oxidationsstatus des Kupferatoms zu regenerieren.

Die Aktivität des Enzyms wird potentiometrisch aufgrund des Verbrauchs von molekularem Sauerstoff bestimmt. Der jeweilige Testansatz enthielt folgende Komponenten:
Je 100 µl Tyramin, Fumarat, Ascorbat (jeweils 5 mM)
1 ml Phosphat-Puffer, pH 5 (100 mM)
1 ml Wasser
100 µl Enzym (entsprechend 0,1 E).

Die Sauerstoff-Verbrauchsraten wurden anhand des $O_2$-Gehaltes einer 37°C Lösung bei jeweiligem Volumen errechnet.

Bei Zugabe von Eschscholtzia-Extrakt zeigt sich eine deutliche, konzentrationsabhängige Hemmung der Enzymreaktion, d.h. die Seitenketten-Hydroxylierung wird weitgehend unterbunden. Es wurde beobachtet, dass die Enzymaktivität auch durch Etbanol gehemmt wird. Doch ist die Hemmung durch den Extrakt wesentlich höher, so dass hier deutlich unterschieden werden kann.

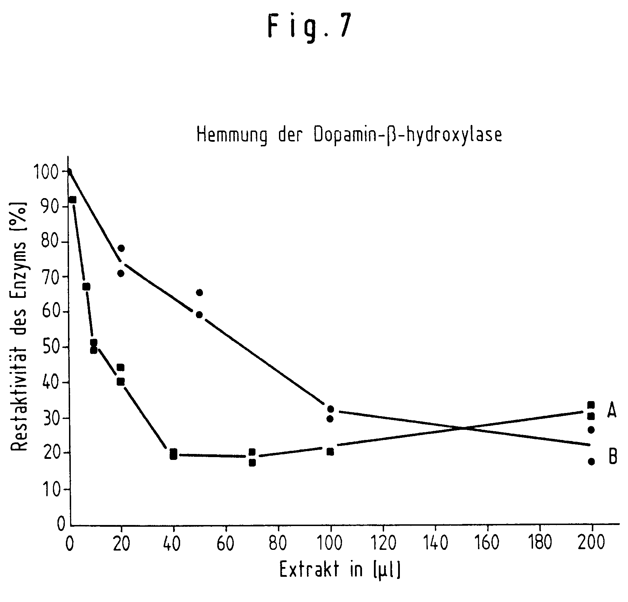

Fig. 7 zeigt die Hemmung der Dopamin-β-hydroxylase durch Eschscholtzia-Extrakt (Kurve A) sowie einer wässrigen 1%-igen Lösung der gefriergetrockneten Extrakte von Eschscholtzia und Corydalis im Verhältnis 20:1 (Kurve B) gemäß der Erfindung.

In Fig. 7 zeigen beide Kurven eine deutliche Hemmung der Enzymaktivität. Unterschiede im Kurvenverlauf dürften auf die An- bzw. Abwesenheit von Ethanol sowie von Corydalis-Extrakt (Kurve B) zurückgehen. Die nachlassende Wirkung der Enzymhemmung beim Eschscholtzia-Extrakt kann zum Teil auf einen Sauerstoff-Verbrauch durch die verschiedenen Substrate des Extraktes zurückgeführt werden. Die wässrige Lösung der gefriergetrockneten Extrakte von Corydalis und Eschscholtzia zeigen zwar ebenfalls einen Sauerstoff-Verbrauch mit Tyramin, Ascorbat und Fumarat; die Hemmwirkung auf das Enzym lässt jedoch nicht nach, wodurch hier eine Interpretation des Effektes erheblich erschwert wird.

Die vorstehenden Versuche zeigen deutlich, dass das Enzym Dopamin-β-hydroxylase durch den alkoholischen Extrakt von Eschscholtzia (Kurve A) sowie auch durch eine wässrige Lösung der gefriergetrockneten Extrakte von Eschscholtzia und Corydalis (Kurve B) gehemmt wird, was im Catecholamin-Stoffwechsel zu einer Verminderung der Noradrenalin- und Adrenalinbildung führt. Diese Hemmwirkung erklärt die sedative Wirkung des erfindungsgemässen Arzneimittels. Die durch die Hemmung aufrechterhaltene hohe Konzentration an Dopamin bewirkt einen positiven Einfluss im Falle von Parkinsonismus, einem Krankheitsbild, in dem dopaminerge Nervenzellen zerstört sind und der Dopamingehalt im Gehirn durch künstliche Gaben hochgehalten werden muss.

## 3. Untersuchung der Monoaminooxidase-Reaktion

Wie aus Fig. 1 ersichtlich ist, wird die Konzentration von Dopa bzw. Dopamin auch durch dessen Abbau infolge von Monoaminooxidasen reguliert. Die Monoaminooxidasen setzen primäre Amine mit Hilfe von Sauerstoff im wässrigen Medium zum entsprechenden Aldehyd, Ammoniak und Wasserstoffperoxid im stöchiometrischen Verhältnis um. Die hier verwendete Aminooxydase wurde von der Firma Sigma Chemicals bezogen. Diese Aminooxidase zeigt eine hohe Affinität zu Benzylamin als Substrat. Daher wurden die nachfolgenden Untersuchungen mit Benzylamin als Substrat durchgeführt und der Einfluss von Corydalis und Eschscholtzia auf die Aktivität dieser Monoaminooxidase geprüft.

Der Umsatz von Benzylamin zu Benzylaldehyd durch die Monoaminooxidase wurde fotometrisch und potentiometrisch nachgewiesen:

a) Photometrisch erfolgt der Nachweis des entstehenden Benzaldehyds, der bei 241 nm einen hohen molekularen Extinktionskoeffizienten $(1,2 \times 10^4)$ aufweist. Dieser empfindliche Nachweis erlaubt den Einsatz von sehr kleinen Enzymmengen, die bei 0,01 und 0,02 E Monoaminooxidase (MAO) in einem Reaktionsvolumen von 2 ml liegen können.

Der jeweilige Testansatz mit 2.000 μl Gesamtvolumen enthielt:
50 μl einer 100 mM wässrigen Benzylamin.HCl-Lösung (entsprechend 2,5 mM),
100 mM Phosphatpuffer, pH 7,4,
0,01 E oder 0,02 E MAO, zugegeben in 100 μl-Portionen einer entsprechenden Stammlösung;
die Referenzküvette enthielt Puffer, Wasser und Benzylamin.

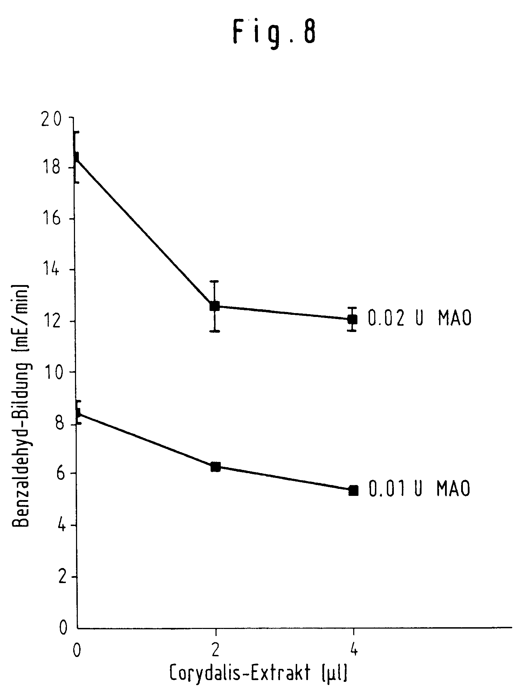

In den Versuchen nit dem oben beschriebenen Testansatz wurden nach 10 Minuten Reaktionszeit in die Mess- und Referenzküvette die verdünnten Extrakte von Eschscholtzia und Corydalis zugegeben und die veränderte Aktivität bestimmt. Fig. 8 zeigt die Hemmung der Benzaldehyd-Bildung in Abhängigkeit von der Corydalis-Konzentration (20 bzw. 40 μl einer 1:10 verdünnten Lösung des Stamm-Extraktes).

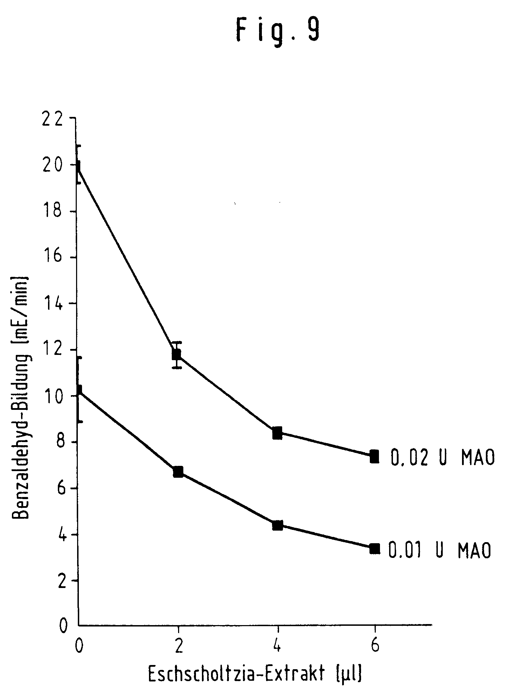

In Fig. 9 wird die Hemmung des Extraktes aus Eschscholtzia auf die MAO-Aktivität bei unterschiedlichen Extraktmengen wiedergegeben. Hierbei zeigt sich eine deutliche Hemmung der MAO-Aktivität.

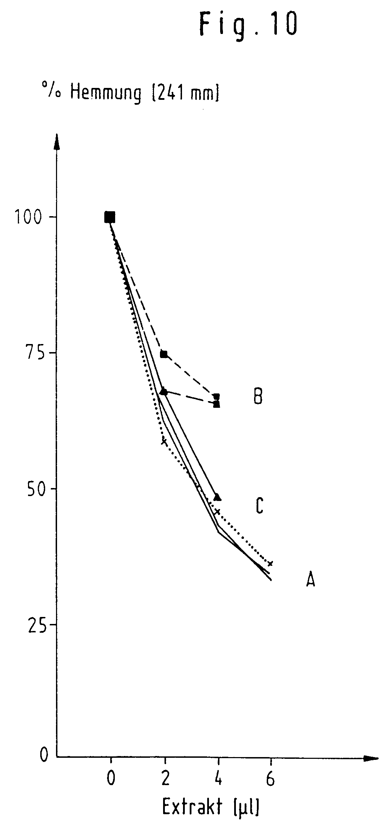

Fig. 10 zeigt die Hemmwirkung der MAO-Aktivität durch Zugabe unterschiedlicher Konzentrationen an Eschscholtzia-Extrakt (Kurve A), Corydalis-Extrakt (Kurve B) sowie einer Mischung der Extrakte von Corydalis und Eschscholtzia im Verhältnis 20:80 (Kurve C). Zum besseren Vergleich der Versuchsergebnisse erfolgte in Fig 10 die Darstellung der Hemmwirkungen in %. Die Aktivität der MAO wurde dabei in jedem Versuch als 100% angesetzt (aufgrund der viskosen Ammoniumsulfat-Enzym-Lösung war es schwierig, konstants Enzymmengen des im Handel angebotenen Produktes zu entnehmen).

Die Auswertung der Versuche zeigt, dass durch den Corydalis-Extrakt eine mässige Hemmung der MAO-Aktivität bewirkt wird. Ein wesentlich stärkerer Einfluss ist durch den Eschscholtzia-Extrakt zu beobachten. Ebenfalls bedeutend ist die MAO-Hemmung in Gegenwart eines Gemisches der beiden Extrakte, bestehend aus 80% Eschscholtzia-Extrakt und 20% Corydalis-Extrakt.

b) Potentiometrisch kann die Reaktion Benzylamin + $O_2$ + $H_2O$ -> Benzaldehyd + $NH_3$ + $H_2O_2$ durch die Abnahme des Sauerstoffgehaltes der Reaktionslösung mit Hilfe einer Platin-Sauerstoffelektrode bestimmt werden. Der Nachweis des Sauerstoff-Umsatzes erfordert höhere Substrat- und Enzymmengen. Der jeweilige Testansatz enthielt:

200 µl einer 100 mM Benzylaminlösung (entsprechend 10 mM)

1.000 µl einer 200 mM Phosphatpufferlösung, pH 7,5 (entsprechend 100 mM),

1.000 µl dest. Wasser,

0,05 E MAO.

Durch Zugabe von 100 µl der Extrakte von Corydalis und Eschscholtzia nach 3 Minuten Reaktionszeit können die unterschiedlichen Raten des Sauerstoffumsatzes errechnet werden.

Die Ergebnisse an der Sauerstoff-Elektrode zeigen eine Aktivitätsminderung der Monoaminoozidase durch die einzelnen Extrakte aus Corydalis und Eschscholtzia. Dabei wurde die starke Beeinflussung der MAO-Aktivität durch den Eschscholtzia-Extrakt bestätigt.

Die vorstehenden Versuche zeigen, dass eine signifikante Hemmung der MAO durch Extrakte aus Corydalis und Eschscholtzia sowie einer Mischung dieser beiden Extrakte erzielt werden kann. Daraus ist abzuleiten, dass wenn Aminooxidasen im Plasma in ihrer Aktivität gehemmt werden, Amine, wie Dopamin, eine längere Verweildauer im Blutstrom haben und damit länger wirksam bleiben. Daraus leitet sich der positive Effekt des erfindungsgemässen Arzneimittels bei der Linderung von Erregungszuständen und Nervendysfunktionen und den sich daraus ableitenden Depressionszuständen, insbesondere auch bei Dopamin-Mangelsyndromen, wie Parkinsonismus, ab.

**BEISPIELE**

**<u>Beispiel 1</u>**

Extraktherstellung

Es wurden Einzelextrakte von Corydalis cavae rhizoma und Eschscholtzia californicae herba hergestellt. Die Herstellung der Einzelextrakte erfolgte durch Extraktion mittels Perkolation bei 50°C während 16 Stunden. Es wurde ein Drogen-Extrakt-Verhältnis von 2.5:10 eingehalten, d.h. aus 2.5 g der entsprechenden Droge wurden 10 ml des Drogenextraktes gewonnen. Extraktionsmittel: 30 Vol. % Ethanol.

Die so erhaltenen Extrakte können direkt zur Herstellung einer Arzneimittel-Zusammensetzung eingesetzt werden, oder sie werden durch Lyophilisation in ein Trockenextrakt übergeführt.

## Beispiel 2

Einfluß von DL-Dihydroxyphenylalanin auf die Phenolase-Reaktion

Reaktionsbedingungen: Phosphatpuffer, pH = 6.5, 100 mM
Tyrosin 2 mM
Phenolase 20 U
DL-Dihydroxyphenylalanin, variabel

| | Phenolase | | + DOPA 500 nM | | + DOPA 5 µM | | + DOPA 50 µM | |
|---|---|---|---|---|---|---|---|---|
| t (min) | mE | +/- | mE | +/- | mE | +/- | mE | +/- |
| 0 | 0 | 0 | 0 | 0 | 0 | 0 | 0 | 0 |
| 2 | | | 4 | 0 | 23 | 2.2 | 70 | 4.6 |
| 4 | | | 13 | 1.6 | 69 | 2.9 | 157 | 6.1 |
| 6 | 5 | 0.63 | 28 | 3.2 | 117 | 5.2 | 236 | 11 |
| 8 | 6 | 1.2 | 50 | 2.9 | 170 | 4.7 | 321 | 15 |
| 10 | 12 | 1.5 | 75 | 5.1 | 223 | 7.2 | 402 | 15 |
| 12 | 23 | 2.2 | .. | .. | .. | .. | .. | .. |
| 14 | 39 | 3.1 | | | | | | |
| 16 | 60 | 4.7 | | | | | | |
| 18 | 84 | 7.3 | | | | | | |
| 20 | 139 | 18 | 291 | 14 | 554 | 16 | 775 | 29 |
| .. | .. | .. | .. | .. | .. | .. | .. | .. |
| 30 | 404 | 32 | 562 | 23 | 883 | 21 | 1090 | 37 |

Wie Fig. 6 zeigt, kann DL-Dopa die lag-Phase der Phenolase deutlich verkürzen, wobei 5 µM Dopa bereits eine sofortige Dopachrombildung auslösen. Mit 50 µM Dopa läuft die Reaktion unmittelbar nach dem Start durch das Enzym schon mit maximaler Umsatzrate.

Die Autoxidation von Dopa, also eine Dopachrombildung ohne Enzymbeteiligung, ist bei pH=6.5 im beobachteten Zeitraum nicht feststellbar.

DL-Dopa stellt jedoch nicht ein beliebiges Diol, sondern ein Substrat der Phenolase dar, dessen Produkt wiederum Dopachrom ergibt. Deshalb werden nachfolgend (Beispiel 3) vergleichende Untersuchungen mit 3,4 Dihydroxybenzol unternommen.

8

## Beispiel 3

Einfluß von 3,4-Dihydroxybenzol (als Starter) auf die Phenolasereaktion

Reaktionsbedingungen: Phosphatpuffer, pH = 6.5, 100 mM
Tyrosin 2 mM
Phenolase 20 U
3,4-Dihydroxibenzol, variabel

| t (min) | Phenolase mE | +/- | + 3,4-DHB 5 µM mE | +/- | + 3,4-DHB 50 µM mE | +/- |
|---|---|---|---|---|---|---|
| 0 | 0 | 0 | 0 | 0 | 0 | 0 |
| 2 | | | 6 | 1.7 | 27 | 2.7 |
| 4 | | | 15 | 1.3 | 63 | 4.8 |
| 6 | 8 | 0.91 | 33· | 4.1 | 102 | 8.9 |
| 8 | 16 | 1.3 | 58 | 6.2 | 143 | 13 |
| 10 | 38 | 2.7 | 97 | 11 | 180 | 18 |
| .. | .. | .. | .. | .. | .. | .. |
| 20 | 312 | 14 | 355 | 42 | 446· | 42 |
| .. | .. | .. | .. | .. | .. | .. |
| 30 | 678 | 29 | 638 | 64 | 712 | 69 |

Es zeigt sich, daß o-Dihydroxybenzol in Konzentrationen von 5 und 50 µ M die lag-Phase der Phenolasereaktion verkürzen bzw aufheben kann (Fig. 5)

## Beispiel 4

a) Ermittlung der Michaelis-Konstante für den Tyrosinumsatz durch die Phenoloxidase

Reaktionsbedingungen: Phosphatpuffer, pH = 6.5, 100 mM
Tyrosin 20 µM bis 2 mM
Phenolase 20 U/Ansatz

| Substrat-<br>konzentration | reziproke<br>Konzentration<br>$1/(M)$ | reziproke<br>Umsatzrate<br>$1/(mE/min)$ |
|---|---|---|
| **Bereich 20 µM bis 1 mM** | | |
| 20 µM | 0.05 | 0.27 |
| 40 µM | 0.025 | 0.173 |
| 60 µM | 0.0167 | 0.0116 |
| 80 µM | 0.0125 | 0.098 |
| 100 µM | 0.01 | 0.076 |
| 500 µM | 0.002 | 0.044 |
| 1 mM | 0.001 | 0.040 |

$$K_M = 141 \ \mu M$$

| | | |
|---|---|---|
| **Bereich 20 bis 70 µM** | | |
| 20 µM | 0.05 | 0.163 |
| 30 µM | 0.033 | 0.125 |
| 40 µM | 0.025 | 0.100 |
| 50 µM | 0.020 | 0.081 |
| 70 µM | 0.014 | 0.061 |

$$K_M = 145 \ \mu M$$

| | | |
|---|---|---|
| **Bereich 100 bis 200 µM** | | |
| 100 µM | 0.01 | 0.0555 |
| 120 µM | 0.0083 | 0.0525 |
| 140 µM | 0.0071 | 0.0475 |
| 160 µM | 0.0063 | 0.0435 |
| 180 µM | 0.0056 | 0.0415 |
| 200 µM | 0.005 | 0.040 |

$$K_M = 142 \ \mu M$$

| | | |
|---|---|---|
| **Bereich 1 bis 2 mM** | | |
| 1000 µM | 0.001 | 0.03352 |
| 1400 µM | 0.00071 | 0.0340 |
| 1800 µM | 0.00055 | 0.0334 |
| 2000 µM | 0.0005 | 0.0330 |

$$K_M = 137 \ \mu M$$

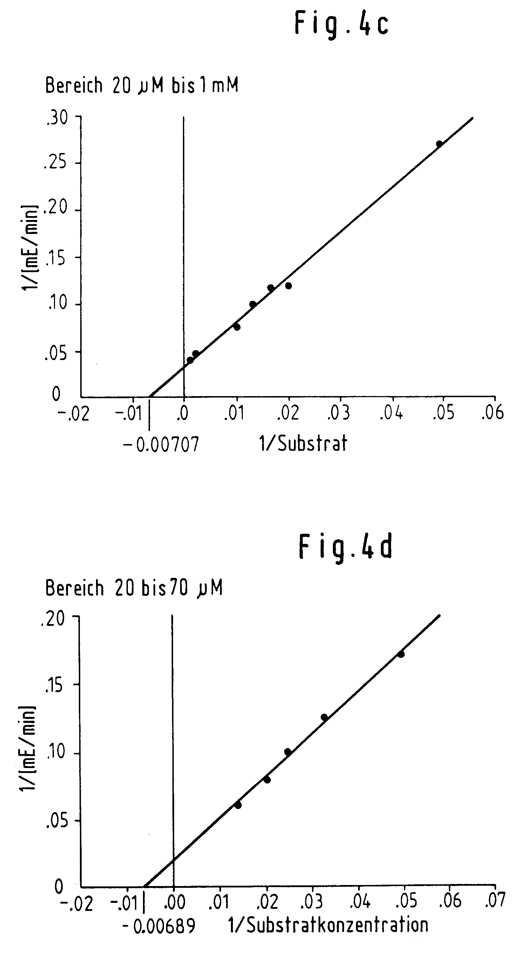

Die Ergebnisse sind in den Fig. 4c bis 4f dargestellt.

## b) **Michaelis-Konstante der Reaktion mit Corydalisextrakt**

Die Untersuchung der Umsatzrate in Anwesenheit des Corydalisextraktes wird mit einer Verdünnung des Extraktes von 1:200 durchgeführt. Diese Extraktmenge führte in den vorangegangenen Versuchen zu einem sofortigen Einsetzen der vollen Enzymaktivität, die Initialphase beträgt 0 Minuten.

Die ermittelten $K_M$-Werte zeigen in Anwesenheit von Corydalisextrakt eine etwas höhere Streuung, liegen jedoch im gleichen Konzentrationsbereich.

Michaeliskonstanten in Anwesenheit von Corydalisextrakt

| Reaktionsbedingungen: Phosphatpuffer, pH = 6.5, 100 mM Tyrosin 20 μM bis 2 mM Phenolase 20 U/Ansatz Corydalis-Extrakt, 1:2000 | | | |
|---|---|---|---|
| Substrat-konzentration | reziproke Konzentration 1/(M) | reziproke Umsatzrate 1/(mE/min) | |
| Bereich 20 bis 100 μM | | | |
| 20 μM | 0.05 | 0.083 | 0.262 |
| 30 μM | 0.033 | 0.057 | |
| 40 μM | 0.025 | 0.045 | |
| 50 μM | 0.020 | 0.040 | |
| 60 μM | 0.017 | 0.033 | 0.110 |
| 70 μM | 0.014 | 0.030 | |
| 100 μM | | | 0.080 |
| $K_M$ = | | 164 μM | 137 μM |
| Bereich 100 bis 200 μM | | | |
| 100 μM | 0.01 | 0.0500 | |
| 120 μM | 0.0083 | 0.0435 | |
| 140 μM | 0.0071 | 0.0414 | |
| 160 μM | 0.0063 | 0.0383 | |
| 180 μM | 0.0056 | 0.0370 | |
| 200 μM | 0.005 | 0.0344 | |
| $K_M$ = | | 151 μM | |

Die Ergebnisse sind in den Fig. 4a und 4b dargestellt.

Beispiel 5

## **Einfluß auf die MAO-Aktivität**

### **a) Einfluß des Corydalis Estraktes:**

Wie die nachfolgende Tabelle und Fig.8 zeigen, hemmt der Extrakt aus Corydalis cava die Aktivität der MAO. Zum Einsatz kommen dabei 20 bzw 40 μl einer 1:10 verdünnten Lösung des Eitrakts (2 bzw. 4 μl der Ursubstanz). Diese Mengen entsprechen, bezogen auf ein Gesamt-Reaktionsvolumen von 2 ml, einer Verdünnung von 1:1000 bzw 1:500.

Die Eigenabsorption des Eztraktes bei 241 nm durch die Anwesenheit zahlreicher aromatischer Verbindungen behindert die Untersuchung von größeren Mengen des Extraktes, da dann ein Nullabgleich von Reaktions-und Referenzküvette nicht mehr möglich ist.

Aktivität der MAO [mE/min] unter Einfluß von Corydalis

```
Reaktionsbedingungen: Phosphatpuffer, pH=7.4, 100 mM
                      Benzylamin 2.5 mM
                      Monoaminoxidase 0.01, 0.02 U/Ansatz
                      Corydalis-Extrakt, 1:10, variabel

              Benzaldehyd (mE/min)

              0.01 U MAO:            0.02 U MAO:

              8.35 ± 0.50 (100%)     18.4 ± 1.00 (100%)

+ 2 µl COR    6.30 ± 0.02 (75.4%)    12.6 ± 1.00 (68.5%)
+ 4 µl COR    5.50 ± 0.00 (65.9%)    12.2 ± 0.45 (66.3%)
```

Die Ergebnisse sind in Fig. 8 dargestellt.

**b) Einfluß des Eschscholtzia Extraktes:**

Aufgrund der höheren Transparenz des Eschscholtzia-Extraktes können in dieser Untersuchung 20, 40 und 60 µl einer 1:10 verdünnten Lösung eingesetzt werden. Im Rezktionsansatz befinden sich dann eine 1:1000, 1:500 oder 1:330 verdünnte Lösung des Eschscholtzia-Extraktes.

Die nachstehende Tabelle und Fig. 9 verdeutlichen die hohe Hemmkapazität dieses Extraktes: Bereits mit einer Verdünnung von 1:500 wird die Aktivität der Monoaminooxidase deutlich unter 50% gehalten, mit einer Verdünnung von 1:330 reduziert sich die Aktivität des Enzyms auf ein Drittel des ursprünglichen Wertes.

Aktivität der MAO unter Einfluß von Eschscholtzia

```
Reaktionsbedingungen: Phosphatpuffer, pH=7.4, 100 mM
                      Benzylamin 2.5 mM
                      Monoaminoxidase 0.01, 0.02 U/Ansatz
                      Eschscholtzia-Extrakt, 1:10, variabel
                      Benzaldehyd (me/min)

           MAO  0.01 U              0.02 U

           10.3+/-1.40 (100%)       20.0+/-0.30 (100%)
2µl ESCH   5.75 / 0.25 (55.5%)      11.3 / 0.55 (59%)
4µl        4.50 / 0.00 (43.7%)      8.50 / 0.30 (42.5%)
6µl        3.50 / 0.00 (33.9%)      7.50 / 0.30 (37.5%)
```

Die Ergebnisse sind in Fig. 9 dargestellt.

**Beispiel 6**

Herstellung einer pharmazeutischen Zubereitung

a. Herstellung einer Tinktur

Zur Herstellung einer Tinktur wurden die nach Beispiel 1 erhaltenen alkolischen Drogenauszüge gemischt, und zwar 20 ml des Drogenauszuges von Corydalis cavae rhizoma und 80 ml des Drogenauszuges von Eschschotzia californicae herba. Es werden 100 ml einer gebrauchsfertigen Tinktur erhalten.

b. Herstellung von Tabletten

Nach Beispiel 1 erhaltene Extrakte aus Corydalis cavae rhizoma und Eschschotzia californicae herba wurden getrocknet (z.B. lyphilisiert), und die Trockenextrakte wurden im Gewichtsverhältnis Eschschotzia/Corydalis = 4/1 zusammen mit pulverisierter Stärke (als pharmazeutische Trägersubstanz) vermischt und in einer Tablettenpresse zu Tabletten (0.5 g, Durchmesser 0.6 cm) verpreßt. Der Anteil an Wirkstoffextrakten in den Tabletten betrug 2 Gew. %.

**Patentansprüche**

**Patentansprüche für folgende Vertragsstaaten : AT, BE, CH, DE, DK, FR, GB, IT, LI, LU, NL, PT, SE**

1. Arzneimittel, dadurch gekennzeichnet, daß es als Wirkstoffe durch alkoholische Extraktion (2.5:10) erhaltene Extrakte von Corydalis und Eschscholtzia, gegebenenfalls neben allgemein üblichen Zusätzen, umfaßt, wobei das Verhältnis (Gewicht) der Extrakte Eschscholtzia zu Corydalis im Bereich vom 20:1 bis 1:1 liegt.

2. Arzneimittel nach Anspruch 1, dadurch gekennzeichnet, daß es die Extrakte einer wässerigen und/oder einer alkoholischen Lösung in Form eines Pulvers enthält.

3. Arzneimittel nach Anspruch 1 oder 2, dadurch gekennzeichnet, daß es die Extrakte in Form eines alkoholischen Drogenauszuges enthält.

4. Arzneimittel nach Anspruch 3, dadurch gekennzeichnet, daß es in Form eines alkoholischen Drogenauszuges vorliegt.

5. Arzneimittel nach einem der Ansprüche 1 bis 4, dadurch gekennzeichnet, daß das Verhältnis der alkoholischen Drogenauszüge von Eschscholtzia und Corydalis 10:1 bis 1:2 beträgt.

6. Arzneimittel nach einem der Ansprüche 1 bis 4, dadurch gekennzeichnet, daß das Verhältnis Eschscholtzia zu Corydalis 4:1, bezogen auf die Drogenextrakte, beträgt.

7. Arzneimittel nach einem der Ansprüche 1 bis 6, dadurch gekennzeichnet, daß es als Wirkstoffe Extrakte von Corydalis cavae rhizoma und Eschscholtziae californicae herba enthält.

8. Arzneimittel nach einem der Ansprüche 1 bis 7, dadurch gekennzeichnet, daß es in einer oralen Darreichungsform vorliegt.

9. Arzneimittel nach Anspruch 8, dadurch gekennzeichnet, daß es in Form von Tabletten, Dragees, Kapseln oder Tinkturen vorliegt.

10. Verwendung eines durch alkoholische Extraktion (2.5:10) erhaltenen Extraktes von Corydalis und Eschscholtzia zur Herstellung eines Arzneimittels zur Linderung bzw. Aufhebung von Erregungszuständen und Nervendysfunktionen, die auf einer Störung des Aminhaushaltes beruhen.

11. Verwendung nach Anspruch 10, wobei die Erregungszustände und Nervendysfunktionen auf einer verminderten Konzentration von Dopa/Dopamin im Stoffwechsel des Patienten beruhen.

12. Verwendung nach Anspruch 10 zur Linderung bzw. Aufhebung von Depressionen.

13. Verwendung nach Anspruch 10 zur Linderung der Beschwerden beim Parkinsonismus.

**EP 0 535 317 B1**

**Patentansprüche für folgende Vertragsstaaten : ES, GR**

1. Verfahren zur Herstellung eines Arzneimittels, dadurch gekennzeichnet, daß man durch alkoholische Extraktion (2.5:10) erhaltene Extrakte von Corydalis und Eschscholtzia, gegebenenfalls neben allgemein üblichen Zusätzen herstellt, wobei das Verhältnis (Gewicht) der Extrakte Eschscholtzia zu Corydalis im Bereich von 20:1 bis 1:1 liegt.

2. Verfahren nach Anspruch 1, dadurch gekennzeichnet, daß man die Extrakte einer wässerigen und/oder einer alkoholischen Lösung in die Form des Pulvers bringt.

3. Verfahren nach Anspruch 1 oder 2, dadurch gekennzeichnet, daß man die Extrakte in Form eines alkoholischen Drogenauszuges herstellt.

4. Verfahren nach Anspruch 3, dadurch gekennzeichnet, daß man das Arzneimittel als alkoholischen Drogenauszug herstellt.

5. Verfahren nach einem der Ansprüche 1 bis 4, dadurch gekennzeichnet, daß das Verhältnis der alkoholischen Drogenauszüge von Eschscholtzia und Corydalis 10:1 bis 2:1 beträgt.

6. Verfahren nach einem der Ansprüche 1 bis 4, dadurch gekennzeichnet, daß das Verhältnis Eschscholtzia zu Corydalis 4:1 bezogen auf die Drogenextrakte, beträgt.

7. Verfahren nach einem der Ansprüche 1 bis 6, dadurch gekennzeichnet, daß die Extrakte solche von Corydalis cavae rhizoma und Eschscholtziae carlifornicae herba sind.

8. Verfahren nach einem der Ansprüche 1 bis 7, dadurch gekennzeichnet, daß man eine orale Darreichungsform herstellt.

9. Verfahren nach Anspruch 8, dadurch gekennzeichnet, daß man Tabletten, Dragees, Kapseln oder Tinkturen herstellt.

10. Verwendung eines durch alkoholische Extraktion (2.5:10) erhaltenen Extraktes von Corydalis und Eschscholtzia zur Herstellung eines Arzneimittels zur Linderung bzw. Aufhebung von Erregungszuständen und Nervendysfunktionen, die auf einer Störung des Aminhaushaltes beruhen.

11. Verwendung nach Anspruch 10, wobei die Erregungszustände und Nervendysfunktionen auf einer verminderten Konzentration von Dopa/Dopamin im Stoffwechsel des Patienten beruhen.

12. Verwendung nach Anspruch 10 zur Linderung bzw. Aufhebung von Depressionen.

13. Verwendung nach Anspruch 10 zur Linderung der Beschwerden beim Parkinsonismus.

**Claims**

**Claims for the following Contracting States : AT, BE, CH, DE, DK, FR, GB, IT, LI, LU, NL, PT, SE**

1. Drug, characterized in that it comprises as active agents extracts of Corydalis and Ezchzcholtzia obtained by alcoholic extraction (2.5:10), in addition if need be to commonly used additives, the ratio (weight) of the extracts of Ezchscholtzia to Corydalis lying in the range of 20:1 to 1:1.

2. Drug according to claim 1, characterized in that it contains the extracts of an aqueous and/or of an alcoholic solution in the form of a powder.

3. Drug according to claim 1 or 2, characterized in that it contains the extracts in the form of an alcoholic drug extract.

4. Drug according to claim 3, characterized in that it exists in the form of an alcoholic drug extract.

5. Drug according to one of the claims 1 to 4, characterized in that the ratio of the alcoholic drug extracts of Eschscholtzia and Corydalis amounts to 10:1 to 2:1.

14

6. Drug according to one of the claims 1 to 4, characterized in that the ratio of Eschscholtzia to Corydaliz amounts to 4:1 with reference to the drug extracts.

7. Drug according to one of the claims 1 to 6, characterized in that it contains as active agents extracts of Corydalis cavae rhizoma and Ezchscholtziae californicae herba.

8. Drug according to one of the claims 1 to 7, characterised in that it exists in a form for administering by mouth.

9. Drug according to claim 8, characterised in that it exists in the form of tablets, dragees, capsules or tinctures.

10. Use of an extract of Corydalis and Eschscholtzia obtained by alcoholic extraction (2.5:10) for the production of a drug for the relief or suspension of states of agitation and nervous disorders which are due to disruption of the amine balance.

11. Use according to claim 10, wherein the states of agitation and nervous disorders are due to a reduced concentration of dopa/dopamine in the metabolism of the patient.

12. Use according to claim 10 for the relief or suspension of depressions.

13. Use according to claim 10 for the relief of the ailments in Parkinsonism.

**Claims for the following Contracting States : ES, GR**

1. Method for the production of a drug, characterised in that extracts of Corydalis and Eschscholtzia obtained by alcoholic extraction (2.5:10), in addition if need be to commonly used additives, are produced, the ratio (weight) of the extracts of Eschscholtzia to Corydalis lying in the range of 20:1 to 1:1.

2. Method according to claim 1, characterized in that the extracts of an aqueous and/or of an alcoholic solution are converted to the form of powder.

3. Method according to claim 1 or 2, characterized in that the extracts are produced in the form of an alcoholic drug extract.

4. Method according to claim 3, characterized in that the drug is produced as an alcoholic drug extract.

5. Method according to one of the claims 1 to 4, characterized in that the ratio of the alcoholic drug extracts of Ezchscholtzia and Corydalis amounts to 10:1 to 2:1.

6. Method according to one of the claims 1 to 4, characterised in that the ratio of Eschscholtzia to Corydalis amounts to 4:1 with reference to the drug extracts.

7. Method according to one of the claims 1 to 6, characterized in that the extracts are those of Corydalis cavae rhizoma and Eschscholtziae californicae herba.

8. Method according to one of the claims 1 to 7, characterized in that a form for administering by mouth is produced.

9. Method according to claim 8, characterized in that tablets, dragees, capsules or tinctures are produced.

10. Use of an extract of Corydalis and Eschscholtzia obtained by alcoholic extraction (2.5:10) for the production of a drug for the relief or suspension of states of agitation and nervous disorders which are due to disruption of the amine balance.

11. Use according to claim 10, wherein the states of agitation and nervous disorders are due to a reduced concentration of dopa/dopamine in the metabolism of the patient.

12. Use according to claim 10 for the relief or suspension of depressions.

13. Use according to claim 10 for the relief of the ailments in Parkinsonism.

**Revendications**

**Revendications pour les Etats contractants suivants : AT, BE, CH, DE, DK, FR, GB, IT, LI, LU, NL, PT, SE**

1. Médicament, caractérisé en ce qu'il comprend comme substances actives des extraits obtenus par extraction alcoolique (2,5:10) de Corydalis et d'Eschscholtzia, le cas échéant avec des additifs généralement utilisés, le rapport (pondéral) des extraits d'Eschscholtzia aux extraits de Corydalis étant dans le domaine de 20:1 à 1:1.

2. Médicament selon la revendication 1, caractérisé en ce qu'il contient des extraits d'une solution aqueuse et/ou alcoolique sous la forme d'une poudre.

3. Médicament selon les revendications 1 ou 2, caractérisé en ce qu'il contient des extraits sous la forme d'une macération alcoolique des drogues.

4. Médicament selon la revendication 3, caractérisé en ce qu'il est présent sous la forme d'une macération alcoolique des drogues.

5. Médicament selon l'une des revendications 1 à 4, caractérisé en ce que le rapport des extraits alcooliques des drogues d'Eschscholtzia et Corydalis est de 10:1 à 1:2.

6. Médicament selon l'une des revendications 1 à 4, caractérisé en ce que le rapport d'Eschscholtzia à Corydalis est de 4:1 en ce qui concerne les extraits de drogues.

7. Médicament selon l'une des revendications 1 à 6, caractérisé en ce qu'il contient comme substances actives des extraits de Corydalis cavae rhizoma et d'Eschscholtziae californicae herba.

8. Médicament selon l'une des revendications 1 à 7, caractérisé en ce qu'il est présent dans une présentation orale.

9. Médicament selon la revendication 8, caractérisé en ce qu'il est présent sous la forme de comprimés, de dragées, de capsules ou de teintures.

10. Utilisation d'un extrait obtenu par extraction alcoolique (2,5:10) de Corydalis et d'Eschscholtzia pour la préparation d'un médicament pour le soulagement ou la suppression des états d'agitation et des dysfonctionnements nerveux qui proviennent d'une perturbation de l'économie des amines.

11. Utilisation selon la revendication 10, dans laquelle les états d'agitation et les dysfonctionnements nerveux proviennent d'un abaissement de la concentration de la dopa/dopamine dans le métabolisme du patient.

12. Utilisation selon la revendication 10, pour le soulagement ou la suppression de dépressions.

13. Utilisation selon la revendication 10, pour le soulagement des douleurs dans la maladie de Parkinson.

**Revendications pour les Etats contractants suivants : ES, GR**

1. Procédé de préparation d'un médicament, caractérisé en ce qu'on prépare des extraits obtenus par extraction alcoolique (2,5:10) de Corydalis et d'Eschscholtzia, le cas échéant avec des additifs généralement utilisés, le rapport (pondéral) des extraits d'Eschscholtzia aux extraits de Corydalis étant dans le domaine de 20:1 à 1:1.

2. Procédé selon la revendication 1, caractérisé en ce qu'on amène les extraits d'une solution aqueuse et/ou alcoolique sous la forme d'une poudre.

3. Procédé selon les revendications 1 ou 2, caractérisé en ce qu'on prépare les extraits sous la forme d'une macération alcoolique de drogues.

4. Procédé selon la revendication 3, caractérisé en ce qu'on prépare le médicament sous la forme d'une macération alcoolique de drogues.

5. Procédé selon l'une des revendications 1 à 4, caractérisé en ce que le rapport des extraits alcooliques de drogues d'Eschscholtzia et de Corydalis est de 10:1 à 2:1.

**6.** Procédé selon l'une des revendications 1 à 4, caractérisé en ce que le rapport d'Eschscholtzia à Corydalis est de 4:1 par rapport aux extraits de drogues.

**7.** Procédé selon l'une des revendications 1 à 6, caractérisé en ce que les extraits sont des extraits de Corydalis cavae rhizoma et d'Eschscholtziae californicae herba.

**8.** Procédé selon l'une des revendications 1 à 7, caractérisé en ce qu'on prépare présentation orale.

**9.** Procédé selon la revendication 8, caractérisé en ce que l'on prépare des comprimés, des dragées, des capsules ou des teintures.

**10.** Utilisation d'un extrait obtenu par extraction alcoolique (2,5:10) de Corydalis et d'Eschscholtzia pour la préparation d'un médicament pour le soulagement ou la cessation d'états d'excitation et de dysfonctionnements nerveux, qui reposent sur une perturbation de l'économie des amines.

**11.** Utilisation selon la revendication 10, dans laquelle les états d'excitation et les dysfonctionnements nerveux reposent sur un abaissement de la concentration de dopa/dopamine dans le métabolisme.

**12.** Utilisation selon la revendication 10, pour le soulagement ou la cessation des dépressions.

**13.** Utilisation selon la revendication 10, pour le soulagement des douleurs dans la maladie de Parkinson.

# F i g . 1

Übersicht des Catecholamin-Stoffwechsels:

Phenoloxidase

M A O    →  Aldehyd + NH₃
            + H₂ O₂

Dopamin-β-hydroxilase

# Fig. 2

Tyrosin

1. Hydroxylierungsschritt ["Tyrosinase"]

Dopa

2. Oxidationsschritt ["Katecholoxidase"]

Dopachinon

Ringschluß

Dopachrom → Polymerisation zu Melanin

Chinonimin

Spontan -$CO_2$

Dopachrom-Tautomerase

Dihydroxyindol

Dihydroxy-indo lylcarboxylsäure

# Fig.3

Tyrosinhydroxylierung bei 303 nm

Corydalis-Extrakt 1:10.000

Eschscholtzia-Extrakt 1:10.000

Phenolase allein [20µ]

# Fig.4a

Bereich 20 bis 100 µM

# Fig.4b

Bereich 100 bis 200 µM

# Fig. 4c

Bereich 20 μM bis 1 mM

# Fig. 4d

Bereich 20 bis 70 μM

# Fig. 4e

Bereich 100 bis 200 µM

# Fig. 4f

Bereich 1 bis 2 mM

# Fig. 5

Phenolasereaktion mit 3,4-Dihydroxibenzol

# Fig. 6

# Fig. 7

Hemmung der Dopamin-β-hydroxylase

# Fig. 8

# Fig. 9

# Fig.10